# EUROPÄISCHE PATENTSCHRIFT

(11) **EP 0 280 157 B1**
(45) Veröffentlichungstag und Bekanntmachung des Hinweises auf die Patenterteilung: **27.09.1995**
(21) Anmeldenummer: 88102230.5
(22) Anmeldetag: 16.02.1988
(51) Int. Cl.: C07D 501/46, C07D 501/04, A61K 31/545

(54) **Kristallisierte Cephem-Säureadditionssalze und Verfahren zu ihrer Herstellung**
Crystalline addition salts of cephem compounds and process for their preparation
Sels d'addition cristallins de dérivés céphem et procédé pour leur préparation

(30) Priorität: 25.02.1987 DE 3706020
(43) Veröffentlichungstag der Anmeldung: 31.08.1988
(73) Patentinhaber: HOECHST AKTIENGESELLSCHAFT, 65926 Frankfurt am Main (DE)
(72) Erfinder: Lattrell, Rudolf, Dr., D-6240 Königstein/Taunus (DE); Dürckheimer, Walter, Dr., D-6234 Hatterheim am Main (DE); Kirrstetter, Reiner, Dr., D-6233 Kelkheim (Taunus) (DE)

(56) Entgegenhaltungen:
- EP-A- 0 064 740
- EP-A- 0 112 550
- EP-A- 0 157 538
- FR-A- 2 466 469

## Beschreibung

Gegenstand der Erfindung ist ein kristallisiertes Säureadditionssalz eines Antibiotikums der Formel I sowie das Verfahren zur Herstellung dieser Verbindung 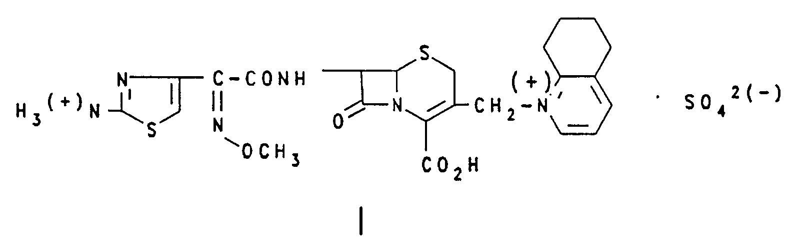
Das Sulfat der Formel I ist für die parenterale Anwendung ganz besonders geeignet, da es sich durch eine sehr geringe Tendenz des Einbaus organischer Lösungsmittel in das Kristallgitter auszeichnet.

Das Verfahren zur Herstellung des Säureadditionssalzes der Formel I ist dadurch gekennzeichnet, daß man ein Cephem-Betain der Formel II 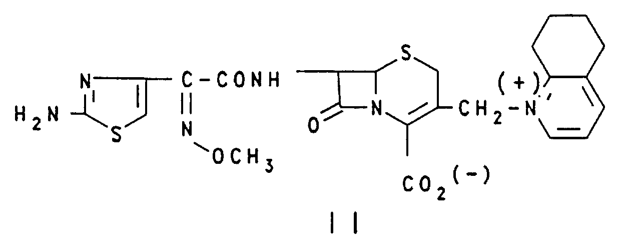
mit Schwefelsäure umsetzt.

Die Herstellung des den erfindungsgemäßen Salzen der allgemeinen Formel I zugrundeliegenden Betains der Formel II wird in dem Europäischen Patent Nr. 0 064 740 (Beispiel 51) beschrieben.

Zur Isolierung des Betains der Formel II aus den Reaktionsansätzen ist besonders das Dihydroiodid des Betains geeignet.

In dem Verfahren zur Herstellung des Salzes der Formel I wird eine Lösung der Verbindung II in Wasser oder in einem Gemisch aus Wasser und einem mit Wasser mischbaren organischen Lösungsmittel, wie z.B. Methanol, Ethanol, Isopropanol, Aceton, Tetrahydrofuran oder Acetonitril, mit Schwefelsäure versetzt.

Die Bildung des Salzes der Formel I wird bei Temperaturen zwischen -20 und +80°C, vorzugsweise zwischen -5 und +30°C vorgenommen. Die Kristallisation des Salzes erfolgt spontan beim Stehen, unter Rühren oder nach Animpfen oder durch Ausfällen mit einem Lösungsmittels wie Aceton oder Ethanol.

Die Schwefelsäure muß in mindestens doppelt äquimolarer Menge zugefügt werden, jedoch kann auch ein Überschuß eingesetzt werden.

Nach Zugabe der Schwefelsäure bildet sich zunächst eine Lösung, die filtriert werden kann, wobei es in manchen Fällen von Vorteil sein kann, vor der Filtration mit Aktivkohle zu klären.

Das Verfahren kann auch so durchgeführt werden, daß man die Schwefelsäure vorlegt und die Cephembase II direkt oder in Wasser gelöst, zugibt.

Die Ausgangsverbindung der Formel II kann auch aus einem ihrer Salze durch Behandeln mit einem basischen Ionenaustauscher, z.B. dem Festaustauscher Amberlite IRA 93 oder dem Flüssigaustauscher Amberlite LA-2, in wäßriger Lösung freigesetzt werden und dann wie vorstehend in ein Säureadditionssalz überführt werden.

Die nach dem vorstehenden Verfahren erhaltene Verbindung der Formel I wird beispielsweise durch Filtration oder Zentrifugieren isoliert und zweckmäßigerweise mit Hilfe eines wasserentziehenden Mittels, wie z.B. konzentrierter Schwefelsäure oder Phosphorpentoxid, bei Normaldruck oder im Vakuum getrocknet. Hierbei fällt die Verbindung der Formel I in Abhängigkeit von den Trocknungsbedingungen als Hydrat oder wasserfrei an.

Die erfindungsgemäß erhaltene Verbindung der Formel I zeigt bemerkenswert gute antibakterielle Wirksamkeit, sowohl gegen grampositive als auch gramnegative bakterielle Keime.

Auch gegen penicillinase- und cephalosporinase-bildende Bakterien ist die Verbindung der Formel I unerwartet gut wirksam. Da sie zudem günstige toxikologische und pharmakologische Eigenschaften zeigt, stellt sie ein wertvolles Chemotherapeutikum dar.

Die Erfindung betrifft somit auch Arzneipräparate zur Behandlung von mikrobiellen Infektionen bei Säugern (sowohl bei Menschen als auch bei Tieren), die durch einen Gehalt der erfindungsgemäßen Verbindung charakterisiert sind.

Das erfindungsgemäße Produkt kann auch in Kombination mit anderen Wirkstoffen, beispielsweise aus der Reihe der Penicilline, Cephalosporine oder Aminoglykoside zur Anwendung kommen.

Die Verbindung der Formel I kann subkutan, intramuskulär, intraarteriell oder intravenös verabfolgt werden, bei Tieren auch noch intratracheal oder lokal, wie beispielsweise in das Euter von milchgebenden Tieren.

Arzneipräparate, die Verbindung der Formel I als Wirkstoff enthalten, können hergestellt werden, indem man die Verbindungen der Formel I mit einem oder mehreren pharmakologisch verträglichen Trägerstoffen oder Verdünnungsmittel, wie z.B. Puffersubstanzen vermischt, und in eine zur parenteralen Applikation geeignete Zubereitungsform bringt.

Als Verdünnungsmittel seien beispielsweise erwähnt Polyglykole, Ethanol und Wasser. Puffersubstanzen sind beispielsweise organische Verbindungen, wie z.B. N',N'-Dibenzylethylendiamin, Diethanolamin, Ethylendiamin, N-Methylglucymin, N-Benzylphenethylamin, Diethylamin, Tris(hydroxymethyl)-aminomethan, oder anorganische Verbindungen, wie z.B. Phosphatpuffer, Natriumbicarbonat, Natriumcarbonat. Für die parenterale Applikation kommen vorzugsweise Suspensionen oder Lösungen in Wasser mit oder ohne Puffersubstanzen in Betracht.

Geeignete Dosen der Verbindung der allgemeinen Formel I liegen bei der Verabreichung an Menschen bei etwa 0,4 bis 20 g/Tag, vorzugsweise bei 0,5 bis 4 g/Tag für einen Erwachsenen von etwa 60 kg Körpergewicht.

Es können Einzel- oder im allgemeinen Mehrfachdosen verabreicht werden, wobei die Einzeldosis den Wirkstoff in einer Menge von etwa 50 bis 1000 mg, vorzugsweise von etwa 1200 bis 500 mg enthalten kann.

Bei einer Verabreichung an Tiere kommen prinzipiell alle Säugetiere in Betracht, im Hinblick auf ihre Bedeutung insbesondere Haus- und Nutztiere. Die Dosierung variiert bei den einzelnen Tierarten und kann beispielsweise zwischen etwa 2,5 und 150, vorzugsweise zwischen etwa 5 und 50 mg/kg Körpergewicht des Tieres liegen.

Es war überraschend, daß das erfindungsgemäß erhaltene kristalline Säureadditionsprodukt eine unerwartet hohe Steigerung der Stabilität sowohl gegenüber dem Betain als auch entsprechenden amorphen Salzen aufweisen würde. So bleibt bei dem kristallinen Sulfat bei mehrwöchiger Temperaturbelastung die Aktivität fast erhalten, während sie bei den anderen vorstehend genannten Produktformen einen starken Abfall zeigt. Aufgrund dieser unerwartet guten Eigenschaften des Endprodukts weisen die mit ihm hergestellten Zubereitungen, eine weit bessere Reinheit und Sicherheit bei der therapeutischen Anwendung auf.

Die folgenden Ausführungsbeispiele für erfindungsgemäß herstellbare Säureadditionsverbindungen der Verbindung II, 1-[[(6R,7R)(Z)-7-[[2-(2amino-4-thiazolyl)(methoxyimino)acetyl]amino]-2-carboxy-8-oxo-5-thia-1-azabicyclo[4,2,0]oct-2-en-3-yl]methyl]5,6,7,8-tetrahydrochinoliniumhydroxid, inneres Salz, dienen zur weiteren Erläuterung der Erfindung.

### Beispiel 1

### Sulfat der Verbindung II (Verfahren 1)

Eine Mischung aus 8,2 g (0,01 Mol) Dihydrojodidsalz der Verbindung II (Beispiel 2a), 15 ml Amberlite LA-2 (Serva 40610), 30 ml Wasser und 50 ml EtOAc wird 30 Minuten bei 20°C gerührt. Nach 15 Minuten hatte sich das Dihydrojodidsalz gelöst. Die Phasen werden getrennt und die wäßrige Phase wird mit 50 ml Toluol gewaschen. Die wäßrige Phase, die das Betain II enthält, wird nach Zusatz von 0,6 g Tierkohle 15 Minuten gerührt, filtriert, auf 10°C gekühlt und mit 6 N Schwefelsäure auf pH 1,3 angesäuert. Sodann werden unter Rühren 60 ml kaltes Ethanol zugegeben. Nach kurzer Zeit beginnt die Kristallisation des Sulfats. Die Suspension wird 4 Stunden bei 5°C gerührt, filtriert, der Niederschlag zweimal mit je 15 ml Ethanol gewaschen und im Vakuum über P₂O₅ bis zur Gewichtskonstanz getrocknet.
Ausbeute: 5,2 g (80,7 %) farblose Kristalle, Zers. 182°C.

| C₂₃H₂₄N₆O₅S₂ x H₂SO₄ x H₂O (644.7) | | | | | |
|---|---|---|---|---|---|
| Ber.: | C 42,8 | H 4,4 | N 13,1 | S 14,9 | H₂O 2,8 % |
| Gef.: | C 42,6 | H 4,2 | N 13,0 | S 14,7 | H₂O 3,1 % |

### Beispiel 2

### Dihydrojodid der Verbindung II

a) Isolierung von II aus Reaktionsansätzen
22,6 g (0,17 Mol) 5,6,7,8-Tetrahydrochinolin wird unter Rühren zu einer auf 5 C gekühlten Lösung von 28 g (0,14 Mol) Trimethyljodsilan in 200 ml Dichlormethan getropft. In die gerührte Lösung werden sodann 9,1 g (0,02 Mol) 7-[2-(2-Aminothiazol-4-yl)-2-(Z)-methoxyiminoacetamido]-cephalosporansäure eingetragen. Die Mischung wird 2 Stunden unter Rückfluß erhitzt, sodann auf 5°C gekühlt und unter Zutropfen von 60 ml eines Gemisches von 57 % Jodwasserstoffsäure:Wasser:Ethanol (1:4:8) hydrolysiert. Das Dihydrojodidsalz fällt als hellgelber Niederschlag aus. Nach 4 Stunden bei 0°C wird filtriert, mit 100 ml Methylenchlorid und 100 ml Aceton gewaschen und an der Luft getrocknet. Ausbeute: 13 g (79,2 %) hellgelbe Kristalle, Zers. > 200°C

| C₂₃H₂₄N₆O₅S₂ x 2HJ x H₂O (820,5) | | | | | | |
|---|---|---|---|---|---|---|
| Ber.: | C 33,7 | H 3,7 | N 10,2 | S 7,8 | J 30,9 | H₂O 4,3 % |
| Gef.: | C 32,5 | H 3,6 | N 9,8 | S 7,3 | J 30,5 | H₂O 3,8 % |

b) Dihydrojodid der Verbindung II (Verfahren 1)
Zu einer Lösung von 0,01 Mol II, hergestellt nach Beispiel 1, werden 25 ml 1N wäßrige Jodwasserstoffsäure zugegeben. Es bildet sich sofort ein gelber Niederschlag, der abgesaugt, mit Wasser gewaschen und getrocknet wird. Die Verbindung ist in allen Eigenschaften mit der vorstehend beschriebenen identisch.
c) Dihydrojodid der Verbindung II (Verfahren 3)
6,45 g (0,01 Mol) Sulfat (Beispiel 1) werden in 200 ml Wasser gelöst und sodann eine Lösung von 5,5 g (0,035 Mol) Kaliumjodid in 5 ml Wasser zugegeben. Nach kurzer Zeit beginnt die Kristallisation von hellgelbem Dihydrojodidsalz. Nach 2 Stunden wird abgesaugt, mit Wasser gewaschen und getrocknet. Die Verbindung ist in allen Eigenschaften mit der vorstehend beschriebenen identisch.

## Patentansprüche (Patentansprüche für folgende(n) Vertragsstaat(en): AT, BE, CH, DE, FR, GB, IT, LI, LU, NL, SE)

1. Kristallisiertes Cephem-Säureadditionsalz der Formel I

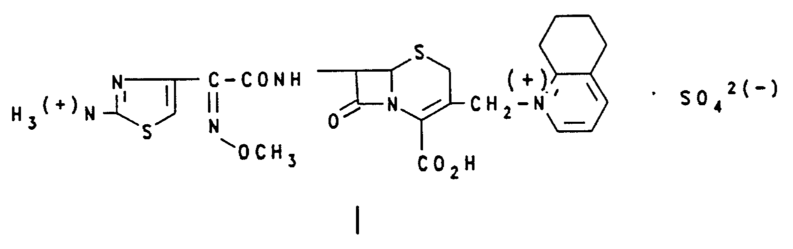

2. Verfahren zur Herstellung des Säureadditionssalzes der Formel I, dadurch gekennzeichnet, daß man ein Cephem-Betain der Formel II 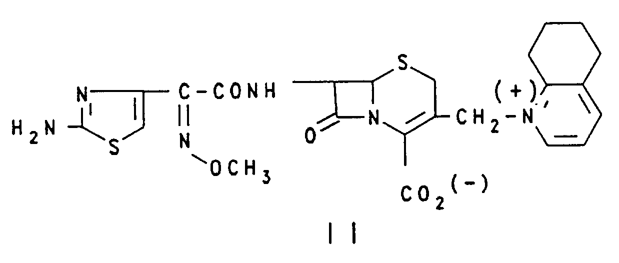 mit Schwefelsäure umsetzt.

3. Arzneimittel, gekennzeichnet durch einen Gehalt an dem Salz der Formel I gemäß Anspruch 1.

4. Verwendung des Salzes der Formel I zur Herstellung eines Arzneimittels, das zur Bekämpfung bakterieller Infektionen geeignet ist.

## Patentansprüche (Patentansprüche für folgende(n) Vertragsstaat(en): ES, GR)

1. Verfahren zur Herstellung von kristallisiertem Cephem-Säureadditionssalz der Formel I 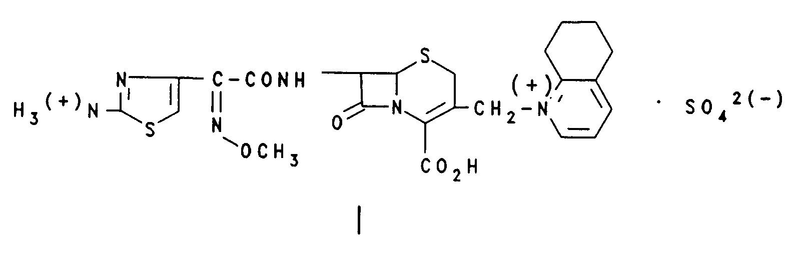 dadurch gekennzeichnet, daß man
ein Cephem-Betain der Formel II 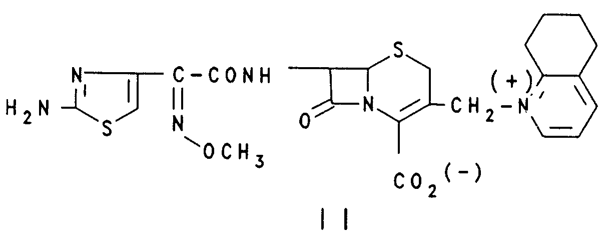 mit Schwefelsäure umsetzt.

2. Verfahren zur Herstellung von gegen bakterielle Infektionen bei Säugern wirksamen pharmazeutischen Präparaten, dadurch gekennzeichnet, daß das Cephemderivat der Formel I gegebenenfalls mit pharmazeutisch üblichen Trägerstoffen, Verdünnungsmitteln oder Puffersubstanzen in eine pharmazeutisch geeignete Verabreichungsform gebracht wird.

3. Verwendung des Salzes der Formel I zur Herstellung eines Arzneimittels, das zur Bekämpfung bakterieller Infektionen geeignet ist.

## Claims (Claims for the following Contracting State(s): AT, BE, CH, DE, FR, GB, IT, LI, LU, NL, SE)

1. A crystallized cephem-acid addition salt of the formula I

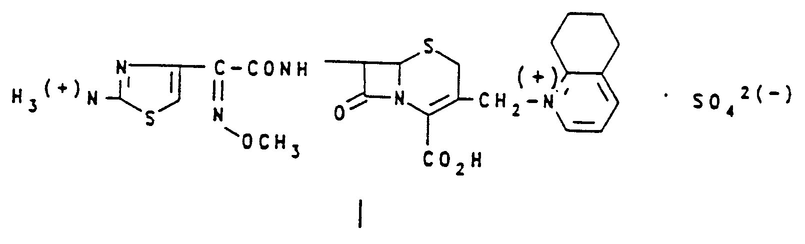

2. A process for the preparation of an acid-addition salt of the formula I, which comprises reacting a cephem-betaine of the formula II 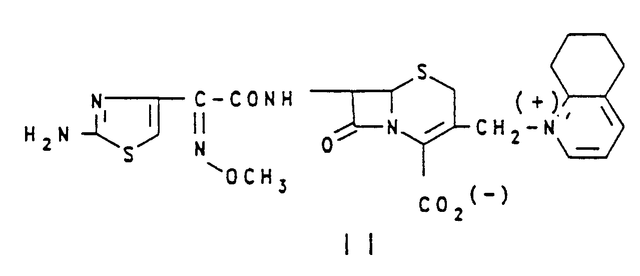 with sulfuric acid.

3. A drug which contains a salt of the formula I as claimed in claim 1.

4. The use of a salt of the formula I for the preparation of a drug which is suitable for combating bacterial infections.

## Claims (Claims for the following Contracting State(s): ES, GR)

1. A process for the preparation of a crystallized cephem-acid addition salt of the formula I 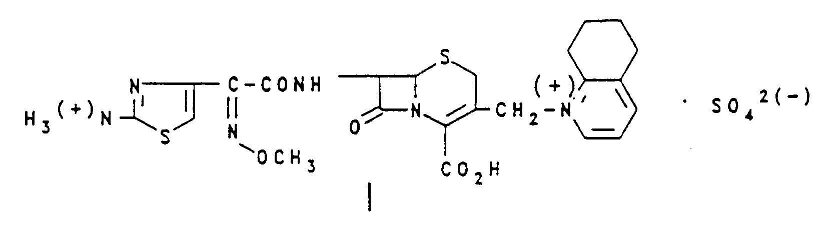 which comprises reacting a cephem-betaine of the formula II 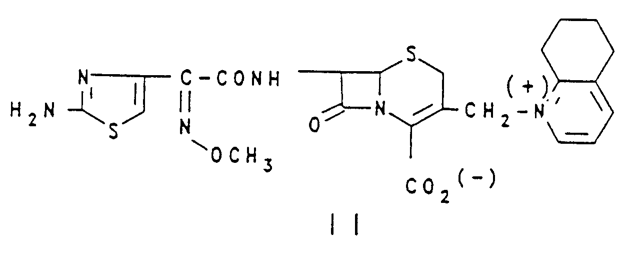 with sulfuric acid.

2. A process for the production of pharmaceutical preparations which are active against bacterial infections in mammals, which comprises converting a cephem derivative of the formula I into a pharmaceutically suitable administration form, with or without pharmaceutically conventional excipients, diluents or buffer substances.

3. The use of a salt of the formula I for the preparation of a drug which is suitable for combating bacterial infections.

## Revendications (Revendications pour l'(les) Etat(s) contractant(s) suivant(s): AT, BE, CH, DE, FR, GB, IT, LI, LU, NL, SE)

1. Sel d'addition avec un acide d'un composé céphème, cristallisé, de formule I

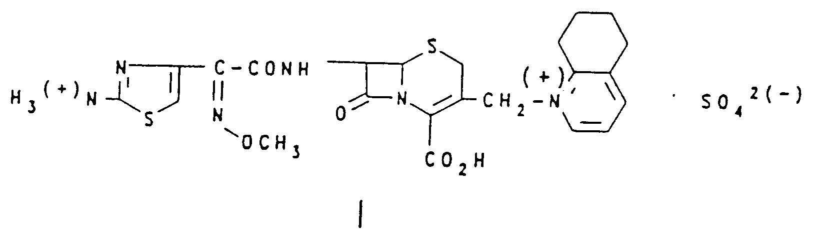

2. Procédé pour la préparation du sel d'addition avec un acide de formule I, caractérisé en ce que l'on fait réagir une céphème-bétaïne de formule II 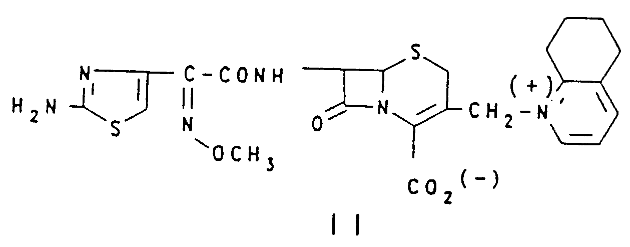 avec de l'acide sulfurique.

3. Médicament caractérisé par une teneur en le sel de formule I selon la revendication 1.

4. Utilisation du sel de formule I pour la fabrication d'un médicament qui est approprié à la lutte contre des infections bactériennes.

## Revendications (Revendications pour l'(les) Etat(s) contractant(s) suivant(s): ES, GR)

1. Procédé pour la préparation d'un sel d'addition avec un acide d'un composé céphème, cristallisé, de formule I 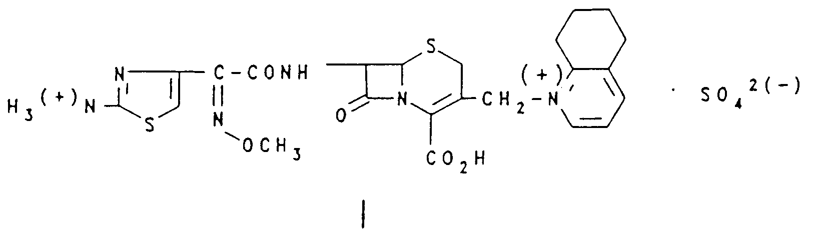 caractérisé en ce que l'on fait réagir une céphème-bétaïne de formule II 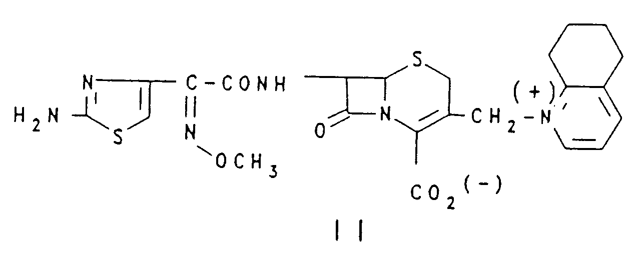 avec de l'acide sulfurique.

2. Procédé pour la préparation de compositions pharmaceutiques actives contre des infections bactériennes chez des mammifères, caractérisé en ce que l'on met sous une forme d'administration pharmaceutiquement appropriée le dérivé céphème de formule I, éventuellement avec des véhicules, diluants ou substances tampons pharmaceutiquement usuelles.

3. Utilisation du sel de formule I pour la fabrication d'un médicament qui est approprié à la lutte contre des infections bactériennes.
